(19) Europäisches Patentamt
European Patent Office
Office européen des brevets

(11) Publication number: **0 028 117**
**B1**

(12) **EUROPEAN PATENT SPECIFICATION**

(45) Date of publication of patent specification: **28.12.83**

(21) Application number: **80303719.1**

(22) Date of filing: **21.10.80**

(51) Int. Cl.³: **C 07 D 417/12,**
A 61 K 31/425
//(C07D417/12, 207/42,
277/48)

(54) **Guanidine compounds, process for preparing them and compositions containing them.**

(30) Priority: **24.10.79 GB 7936810**

(43) Date of publication of application:
**06.05.81 Bulletin 81/18**

(45) Publication of the grant of the patent:
**28.12.83 Bulletin 83/52**

(84) Designated Contracting States:
**AT BE CH DE FR GB IT LI LU NL SE**

(56) References cited:
**DE - A - 2 421 548**
**GB - A - 2 001 624**
**US - A - 4 005 205**

**Burgher's Med. Chemistry, Chapter 48, pp 510 and 511**

(73) Proprietor: **SMITH KLINE & FRENCH LABORATORIES LIMITED**
**Mundells**
**Welwyn Garden City Hertfordshire, AL7 1EY (GB)**

(72) Inventor: **Roantree, Michael Laurence**
**25 Lords Wood**
**Welwyn Garden City Hertfordshire (GB)**
Inventor: **Young, Rodney Christopher**
**62 Cowper Crescent**
**Bengeo Hertfordshire (GB)**

(74) Representative: **Johnston, Walter Paul**
**P.O. Box 39**
**Welwyn Garden City Hertfordshire AL7 1EY (GB)**

**EP 0 028 117 B1**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

# Guanidine compounds, process for preparing them and compositions containing them

This invention relates to guanidine compounds having histamine $H_2$-antagonist activity, their preparation, and pharmaceutical composition containing them.

Histamine, a physiologically active compound endogenous in mammals, exerts its action by interacting with certain sites called receptors. One type of receptor is known as a histamine $H_1$-receptor (Ash and Schild, Brit. J. Pharmac. 1966, 27, 427) and the actions of histamine mediated through these receptors are blocked by drugs commonly called "antihistamines" (histamine $H_1$-antagonists) a common example of which is mepyramine. A second type of histamine receptor is known as the $H_2$-receptor (Black et al. Nature 1972, 236, 385). These receptors are not blocked by mepyramine but are blocked by burimamide. Compounds which block these histamine $H_2$-receptors are called histamine $H_2$-antagonists.

Histamine $H_2$-antagonists are useful in treating disease conditions caused by the biological effects of histamine mediated through $H_2$-receptors, for example, as inhibitors of gastric acid secretion, in the treatment of inflammation mediated through histamine $H_2$-receptors and as agents which act on the cardiovascular system, for example, as inhibitors of effects of histamine on blood pressure mediated through histamine $H_2$-receptors.

Cimetidine is an example of a histamine $H_2$-antagonist. Cimetidine has been shown to be useful in the treatment of duodenal, gastric, recurrent and stomal ulceration, and reflux oesophagitis and in the management of patients who are at high risk from haemorrhage of the upper gastrointestinal tract.

In some physiological conditions the biological actions of histamine are mediated through both histamine $H_1$ and $H_2$-receptors and blockade of both types of receptors is useful. These conditions include inflammation mediated by histamine, for example skin inflammation, and those hypersensitivity responses due to the action of histamine at $H_1$- and $H_2$-receptors, for example allergies.

GB—A—2001624 discloses certain $H_2$-antagonist guanidine-nitroethylene derivatives. In these nitroethylene derivatives, the guanidine-containing moiety can be in cis or trans configuration about the double bond in relation to the nitro group.

A class of guanidine nitropyrrole derivatives have been discovered where the nitro group is fixed in a cis-configuration relative to the guanidine-containing moiety, which are particularly active as histamine $H_2$-antagonists.

Accordingly, the present invention provides compounds of formula (I):—

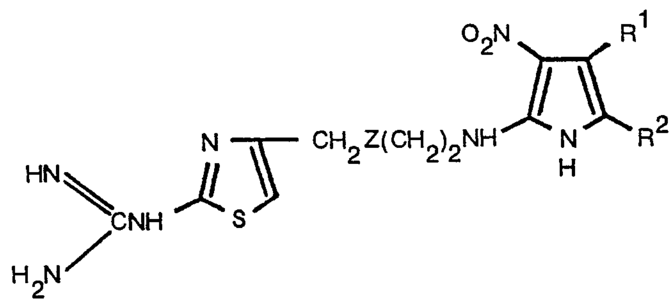

(I)

and pharmaceutically acceptable acid addition salts thereof, where Z is sulphur or methylene and $R^1$ is hydrogen; $C_{1-4}$ alkyl; optionally substituted phenyl or phenyl ($C_{1-4}$ alkyl) (the substituents being one or more $C_{1-4}$ alkyl, or $C_{1-4}$ alkoxy groups or halogen atoms or a methylenedioxy group); or optionally substituted furanyl-, thienyl-, or pyridyl- ($C_{1-4}$ alkyl) (the substituents being one or more $C_{1-4}$ alkyl or $C_{1-4}$ alkoxy groups); and $R^2$ is hydrogen, $C_{1-4}$ alkyl or benzyl.

Examples of substituted phenyl groups and the substituted phenyl moiety of phenyl ($C_{1-4}$ alkyl) groups for $R^1$ are 3-methylphenyl; 3-methoxyphenyl; 3,4-dimethoxyphenyl and 3-chlorophenyl.

Examples of optionally substituted furanyl, thienyl and pyridyl ($C_{1-4}$ alkyl) groups for $R^1$ are optionally substituted 2-furanyl-, 2-thienyl-, 2-pyridyl-, 3-pyridyl-, or 4-pyridyl-($C_{1-4}$ alkyl) groups, and particularly 3-pyridyl-, 6-methyl-3-pyridyl- and 6-methoxy-3-pyridyl-($C_{1-4}$ alkyl)

Preferably $R^1$ is hydrogen.
Preferably $R^2$ is hydrogen.
Preferably Z is sulphur.

Pharmaceutically acceptable acid addition salts of the compounds of formula (I) include those formed with hydrochloric, hydrobromic, hydroiododic, sulphuric and maleic acids.

Preferably the compound is in the form of the free base.

The compounds of the invention can be prepared by reacting a compound of formula (II):—

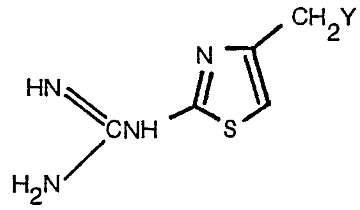

(II)

where Y is either $Z(CH_2)_2NH_2$ or, when Z in the compound of formula (I) is sulphur, a leaving group displaceable by mercaptan, with a compound of formula (III):—

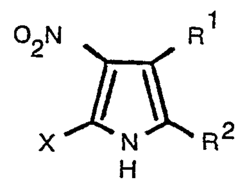

(III)

where X is a leaving group displaceable with an amine when Y is $Z(CH_2)_2NH_2$ or $HS(CH_2)_2NH$— when Y is a leaving group displaceable by mercaptan; and $R^1$ and $R^2$ are as defined with reference to formula (I), and thereafter optionally converting the compound of formula (I) so obtained into a salt.

Examples of leaving groups displaceable by mercaptan are halogen, trisubstituted phosphonium (for example tri-phenylphosphonium) or substituted sulphonyloxy (for example, p-toluenesulphonyloxy, methanesulphonyloxy or trifluoromethanesulphonyloxy).

Examples of leaving groups displacable by amines are where X is QSO— or $QSO_2$— (Q being $C_{1-4}$ alkyl, aryl or aralkyl). Preferably the group X is QSO where Q is methyl or benzyl.

The process where X is a leaving group displaceable by amines is preferably carried out in the presence of a solvent, for example a $C_{1-4}$ alkanol. In general, an elevated temperature will be used, for example, the boiling point of the reaction mixture.

Acid addition salts of compounds of formula (I) can conveniently be formed from the corresponding bases by standard procedures for example by reacting the base with an acid in a $C_{1-4}$ alkanol or by the use of ion exchange resins to form the required salt. Salts of compounds of formula (I) can also be interconverted using an ion exchange resin.

The compounds of formula (I) and the intermediate compounds of formula (III) where X is QSO- or $QSO_2$— can be prepared by reacting a compound of formula (IV):—

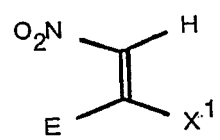

(IV)

wherein E is

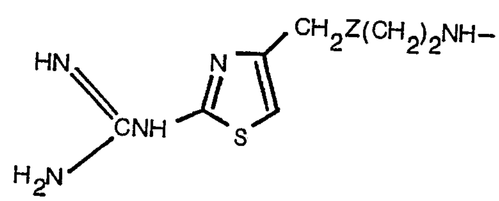

or QS— is $X^1$ is QS, or QSO when E is QS—, with a compound of formula (V):—

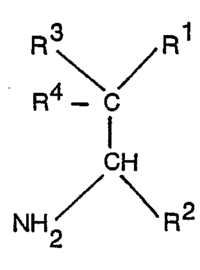

(V)

wherein $R^1$ and $R^2$ are as defined with reference to formula (I) and $R^3$ and $R^4$ are $C_{1-4}$ alkoxy groups or together represent an oxygen atom; optionally converting a compound of formula (I) so obtained into a salt or oxidizing a compound of formula (III) so obtained where X is QS—.

When compound (V) is used, where $R^3$ and $R^4$ together represent an oxygen atom, the reaction will in general be carried out under basic conditions, for example with sodium ethoxide in ethanol. When a compound (V) is used where $R^3$ and $R^4$ are $C_{1-4}$ alkoxy, the reaction is preferably carried out under neutral conditions, for example, in boiling ethanol, and the product cyclised under acidic conditions, for example by treatment with hydrogen chloride in a solvent (for example ether or a $C_{1-4}$ alkanol).

An intermediate (III) obtained by this process where X is QS— can be oxidised with one equivalent of hydrogen peroxide, to an intermediate (III) where X is QSO— and to an intermediate (III) where X is $QSO_2$— by reaction with two or more equivalents of hydrogen peroxide.

The intermediates of formula (III) where X is $HS(CH_2)_2NH$— can be prepared by reacting a compound of formula (III) where X is QSO- or $QSO_2$- with an amine of formula $HS(CH_2)_2NH_2$.

The activity of the compounds of formula (I) as histamine $H_2$-antagonists can be demonstrated by the inhibition of histamine-stimulated secretion of gastric acid from the lumen-perfused stomachs of rats anaesthetised with urethane. This procedure is referred to in Ash and Schild, Brit,. J. Pharmac. Chemother., 1966. 27. 247. The compound of Example 1 hereafter caused 50% inhibition of maximal acid secretion at doses of less than 0.1 micromole $kg^{-1}$ i.v. Their activity as histamine $H_2$-antagonists can also be demonstrated by their ability to inhibit other actions of histamine which, according to the above mentioned paper of Ash and Schild, are not mediated by histamine $H_1$-receptors. For example, they inhibit the actions of histamine on the isolated guinea pig atrium. The potency of these compounds is illustrated by the effective dose producing 50% inhibition of the histamine-induced tachycardia in the isolated guinea pig atrium (less than $10^{-6}$ Molar Example 1).

In order to use compounds of formula (I) or a pharmaceutically acceptable salt thereof for medical purposes, they are normally formulated in accordance with standard pharmaceutical practice as pharmaceutical compositions.

The invention further provides pharmaceutical compositions comprising a compound of formula (I) above or a pharmaceutically acceptable acid addition salt thereof together with a pharmaceutically acceptable carrier.

Compounds of formula (I) and their pharmaceutically acceptable acid addition salts may be administered orally, parenterally, cutaneously or rectally.

Compounds of formula (I) and their pharmaceutically acceptable salts which are active when given orally can be formulated as syrups, tablets, capsules and lozenges. A syrup formulation will generally consist of a suspension or solution of the compound or salt in a suitable liquid carrier for example, ethanol glycerine or water with a flavouring or colouring agent. Where the composition is in the form of a tablet, any suitable pharmaceutical carrier routinely used for preparing solid formulations may be used. Examples of such carriers include magnesium stearate, starch, lactose, sucrose and cellulose.

Typical parenteral compositions consist of a solution or suspension of the compound or salt in a sterile aqueous carrier or parenterally acceptable oil.

Typical compositions for administration to the skin include lotions and creams in which the compound of formula (I) or salt thereof is contained in a liquid vehicle.

A typical suppository formulation comprises a compound of formula (I) or a pharmaceutically acceptable salt thereof which is active when administered in this way, with a binding and/or lubricating agent such as gelatin or cocoa butter or other low melting vegetable waxes or fats.

Preferably the composition is in unit dose form such as a tablet or capsule so that the patient may administer to himself a single dose.

Each dosage unit contains preferably from 15 to 250 mg of a compound of formula (I) or a pharmaceutically acceptable salt thereof calculated as the free base.

The pharmaceutical compositions of the invention will normally be administered to man for the treatment of peptic ulcers and other conditions caused or exacerbated by gastric acidity in the same general manner as that employed for known histamine $H_2$-antagonists, due allowance being made in terms of dose levels for the potency of the compound of the present invention relative to known histamine $H_2$-antagonists. Thus an adult patient will receive an oral dose of between 15 mg and 1500 mg and preferably between 20 mg and 250 mg or an intravenous, subcutaneous or intramuscular dose of between 1.5 mg and 150 mg, and preferably between 5 mg and 20 mg of compound of formula (I) or pharmaceutically acceptable salt thereof calculated as the free base, the composition being administered 1 to 6 times per day.

The following Examples illustrate the invention.

## EXAMPLES

### Example 1

2-[2-(2-Guanidino-4-thiazolylmethylthio)ethylamino]-3-nitropyrrole

A solution of 2-(2-guanidino-4-thiazolylmethylthio)ethamine dihydrochloride (4g, 0.013 mole) in ethanol (25 ml) was basified with a solution of sodium (0.65 g, 0.028 mole) in ethanol (20 ml). The mixture was filtered and the filtrate added to 2-methylsulphinyl-3-nitropyrrole (1.5 g, 0.0086 mole). The mixture was refluxed for 5 days, then the solvent was removed in vacuo and the residence chromatographed on a silica-gel column. The product was eluted with ethyl acetate/propan-2-ol (8:2) and recrystallised from methanol/acetone (6:4). Yield: 0.65 g, 22%. M.P. 195.5—200°C.

### Example 2

2-[2-(2-Guanidino-4-thiazolylmethylthio) ethylamino]-4-methyl-3-nitropyrrole

The title compound is prepared from 2-(2-Guanidino-4-thiazolylmethylthio)-ethylamine dihydrochloride and 2-methylsulphinyl-4-methyl-3-nitropyrrole by a process analogous to that described in Example 1.

### Example 3

2-[4-(2-Guanidino-4-thiazolyl) butylamino]-3-nitropyrrole

The title compound is prepared from 4-(2-guanidino-4-thiazolyl) butylamine dihydrochloride and 2-methylsulphinyl-3-nitropyrrole by a process analogous to that described in Example 1.

**Claims for the Contracting States: BE CH DE FR GB IT LI LU NL SE**

1. A compound for formula (I):—

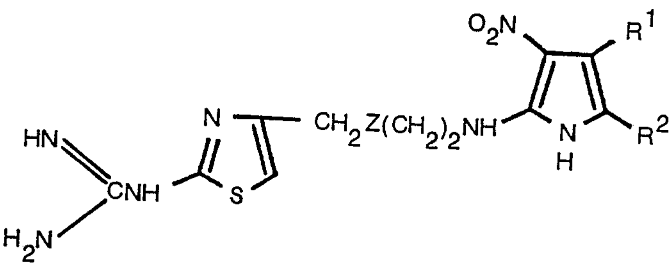

(I)

and pharmaceutically acceptable acid addition salts thereof, where Z is sulphur or methylene and $R^1$ is hydrogen; $C_{1-4}$ alkyl, optionally substituted phenyl or phenyl ($C_{1-4}$ alkyl), (the substituents being one or more ($C_{1-4}$ alkyl or $C_{1-4}$ alkoxy groups or halogen atoms or a methylenedioxo group), or optionally substituted furanyl- or thienyl- or pyridyl- ($C_{1-4}$ alkyl) the substituents being one or more $C_{1-4}$ alkyl or $C_{1-4}$ alkoxy groups; and $R^2$ is hydrogen, $C_{1-4}$ alkyl or benzyl.

2. A compound as claimed in Claim 1 where $R^1$ is 3-methylphenyl, 3-methoxyphenyl; 3,4-dimethoxyphenyl or 3-chlorophenyl.

3. A compound as claimed in Claim 1 or Claim 2 wherein the optionally substituted furanyl-, thienyl- or pyridyl ($C_{1-4}$ alkyl) group $R^1$ is an optionally substituted 2-furanyl-, 2-thienyl-, 3-pyridyl or 4-pyridyl ($C_{1-4}$ alkyl) group.

4. A compound as claimed in any one of Claims 1 to 3 where $R^1$ is 3-pyridyl-, 6-methyl-3-pyridyl-, or 6-methoxy-3-pyridyl ($C_{1-4}$ alkyl).

5. A compound as claimed in any one of Claims 1 to 4 where $R^1$ is hydrogen.

6. A compound as claimed in any one of Claims 1 to 5 where $R^2$ is hydrogen.

7. A compound according to any one of Claims 1 to 6 where Z is sulphur.

8. 2 - [2 - (2 - Guanidino - 4 - thiazolymethylthio) - ethylamino] - 3 - nitropyrrole, 2 - [2 - (2 - Guanidino - 4 - thiazolylmethylthio) - ethylamino] - 4 - methyl - 3 - nitropyrrole and 2 - [2 - (2 - Guanidino - 4 - thiazolylmethylthio)butyl - amino] - 3 - nitropyrrole.

9. A process for preparing a compound as claimed in claim 1 which comprises reacting a compound of formula (II):—

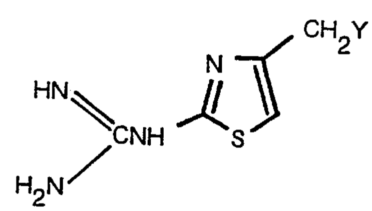

(II)

where Y is either $Z(CH_2)_2NH_2$ or, when Z in the compound of formula (I) is sulphur, a leaving group displaceable by mercaptan, with a compound of formula (III):—

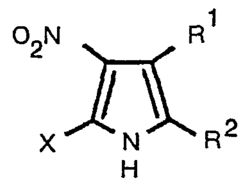

(III)

where X is a leaving group displaceable with an amine when Y is $Z(CH_2)_2NH_2$, or $HS(CH_2)_2NH$— when Y is a leaving group displaceable by mercaptan; and $R^1$ and R are as defined with reference to formula (I), and thereafter optionally converting the compound of formula (I) so obtained into a salt.

10. A process for preparing a compound as claimed in claim 1 which comprises reacting a compound of formula (IV):—

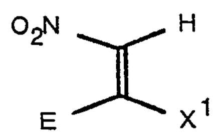

(IV)

where E is

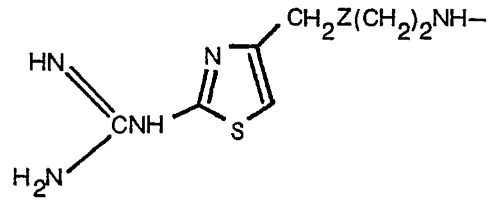

and $X^1$ is a leaving group displaceable by amine of formula QS- (Q being $C_{1-4}$ alkyl, aryl or aralkyl) with a compound of formula (V):—

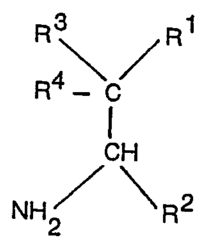

(V)

where $R^1$ and $R^2$ are as defined with reference to formula (I) and $R^3$ and $R^4$ are $C_{1-4}$ alkoxy groups or together represent an oxygen atom; optionally converting a compound of formula (I) so obtained into a salt.

11. A pharmaceutical composition comprising a compound as claimed in anyone of claims 1 to 8 and a pharmaceutically acceptable carrier.

**Claims for the Contracting State: AT**

1. A process for preparing a compound of formula (I):—

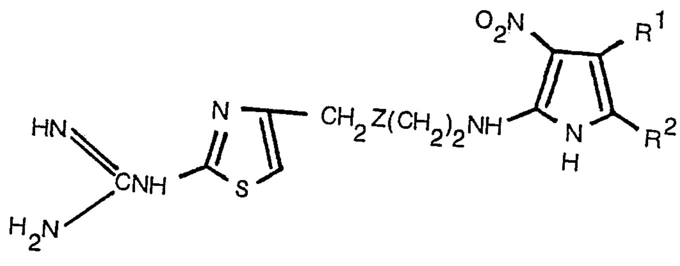

(I)

and pharmaceutically acceptable acid addition salts thereof, where Z is sulphur or methylene and $R^1$ is hydrogen; $C_{1-4}$ alkyl, optionally substituted phenyl or phenyl ($C_{1-4}$ alkyl), (the substituents being one or more $C_{1-4}$ alkyl or $C_{1-4}$ alkoxy groups or halogen atoms or a methylenedioxo group), or optionally substituted furanyl- or thienyl- or pyridyl ($C_{1-4}$ alkyl), the substituents being one or more $C_{1-4}$ alkyl or $C_{1-4}$ alkoxy groups; and $R^2$ is hydrogen, $C_{1-4}$ alkyl or benzyl which comprises either

(a) reacting a compound of formula (II):—

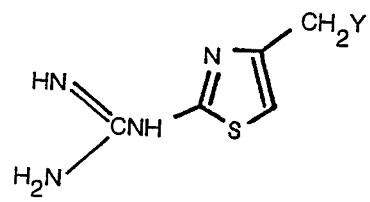

(II)

where Y is either $Z(CH_2)_2NH_2$ or, when Z in the compound of formula (I) is sulphur, a leaving group displaceable by mercaptan, with a compound of formula (III):—

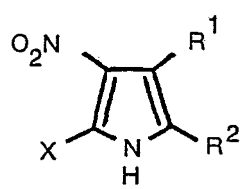

(III)

where X is a leaving group displaceable with an amine when Y is $Z(CH_2)_2NH_2$, or $HS(CH_2)_2NH$— when Y is a leaving group displaceable by mercaptan; and $R^1$ and $R^2$ are as defined with reference to formula (I), or

(b) reacting a compound of formula (IV):—

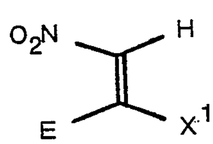

(IV)

where E is

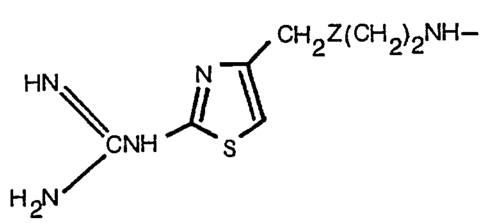

and $X^1$ is a leaving group displaceable by amine of formula QS— (Q being $C_{1-4}$ alkyl, aryl or aralkyl) with a compound of formula (V):—

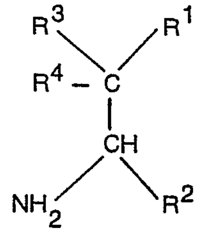

(V)

where $R^1$ and $R^2$ are as defined with reference to formula (I) and $R^3$ and $R^4$ are $C_{1-4}$ alkoxy groups or together represent an oxygen atom; optionally converting a compound of formula (I) so obtained in step (a) or step (b) into a salt.

2. A process as claimed in Claim 1 where $R^1$ in the compound of formula (I) is 3-methyl-phenyl; 3-methoxyphenyl; 3,4-dimethoxyphenyl or 3-chlorophenyl.

3. A process as claimed in Claim 1 or Claim 2 where the optionally substituted furanyl-, thienyl- or pyridyl- ($C_{1-4}$ alkyl) group $R^1$ in the compound of formula (I) produced is an optionally substituted 2-furanyl-, 2-thienyl-3-pyridyl or 4-pyridyl ($C_{1-4}$ alkyl) group.

4. A process as claimed in any one of Claims 1 to 3 wherein $R^1$ in the compound of formula (I) produced is 3-pyridyl-6-methyl-3-pyridyl-, or 6-methoxy-3-pyridyl ($C_{1-4}$ alkyl).

5. A process as claimed in any one of Claims 1 to 4 where $R^1$ in a compound of formula (I) produced is hydrogen.

6. A process as claimed in any one of Claims 1 to 5 in the compound of formula (I) produced in hydrogen.

7. A process according to any one of Claims 1 to 6 where Z in the compound of formula (I) produced is sulphur.

8. A process as claimed in Claim 1 where the product in the compound of formula (I) produced is 2 - [2 - Guanidino - 4 - thiazolylmethylthio) - ethylamino] - 3 - nitropyrrole, 2 - [2 - (2 - Guanidino - 4 - thiazolylmethylthio) - ethylamino]4 - methyl - 3 - nitropyrrole or 2 - [2 - (2 - Guanidino - 4 - thiazolylmethylthio)butylamino] - 3 - nitropyrrole.

**Revendications pour les Etats contractants: BE CH DE FR GB IT LI LU NL SE**

1. Composé de formula (I)

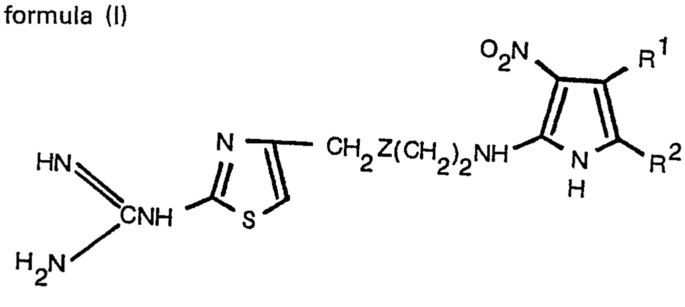

(I)

et ses sels d'addition d'acides pharmaceutiquement acceptables où Z est du soufre ou un groupement méthylène et $R^1$ est de l'hydrogène, un radical alcoyle en $C_{1-4}$, un radical phényle ou phényl ($C_{1-4}$ alcoyle) facultativement substitué (les substituants consistant en un ou plusieurs groupements alcoyle en $C_{1-4}$ ou alcoxy en $C_{1-4}$ ou atomes d'halogène ou un groupement méthylènedioxy) ou un radical furyl- ou thiényl- ou pyridyl- ($C_{1-4}$ alcoyle), les substituants consistant en un ou plusieurs groupements alcoyle en $C_{1-4}$ ou alcoxy en $C_{1-4}$ et $R^1$ est de l'hydrogène, un radical alcoyle en $C_{1-4}$ ou benzyle.

2. Composé tel que revendiqué dans la revendication 1 dans laquelle $R^1$ est un radical 3-méthylphényle, 3-méthoxyphényle, 3,4 diméthoxyphényle ou 3-chlorophényle.

3. Composé tel que revendiqué dans la revendication 1 ou la revendication 2 où le groupement furyl-, thiényl- ou pyridyl- ($C_{1-4}$ alcoyle) facultativement substitué est un groupement 2-furyl-, 2-thiényl-, 3-pyridyl- ou 4-pyridyl- ($C_{1-4}$ alcoyle) facultativement substitué.

4. Composé tel que revendiqué dans l'une quelconque des revendications 1 à 3 où $R^1$ est un radical 3-pyridyl-, 6-méthyl-3-pyridyl- ou 6-méthoxy-3-pyridyl- ($C_{1-4}$ alcoyle).

5. Composé tel que revendiqué dans l'une quelconque des revendications 1 à 4 où $R^1$ est de l'hydrogène.

6. Composé tel que revendiqué dans l'une quelconque des revendications 1 à 5 où $R^2$ est de l'hydrogène.

7. Composé suivant l'une quelconque des revendications 1 à 6 où Z est du soufre.

8. 2 - [2 - (2 - guanidino - 4 - thiazolylméthylthio) - éthylamino] - 3 - nitropyrrole, 2 - [2 - (2 - guanidino - 4 - thiazolylméthylthio) - éthylamino] - 4 - méthyl - 3 - nitropyrrole et 2 - [2 - (2 - guanidino - 4 - thiazolylméthylthio)butylamino - 3 - nitropyrrole.

9. Procédé pour préparer un composé tel que revendiqué dans la revendication 1 qui comprend la réaction d'un composé de formule (II):—

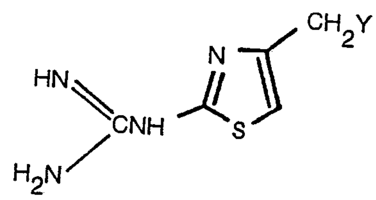
(II)

où Y est soit $Z(CH_2)_2NH_2$ ou, lorsque Z dans le composé de formule (I) est du soufre, un groupe labile déplaçable par un mercaptan, avec un compose de formule (III):—

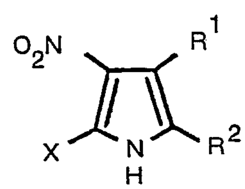
(III)

où X est un groupement labile déplaçable par une amine lorsque Y est $Z(CH_2)_2NH_2$ ou $HS(CH_2)_2NH$— lorsque Y est un groupement labile déplaçable par un mercaptan et $R^1$ et $R^2$ sont tels que définis par référence à la formule (I) et ensuite, facultativement, la conversion du composé de formule (I) ainsi obtenu en un sel.

10. Procédé pour préparer un composé tel que revendiqué dans la revendication 1 qui comprend la réaction d'un composé de formule (IV):—

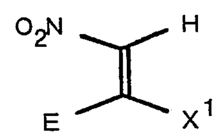
(IV)

où E est

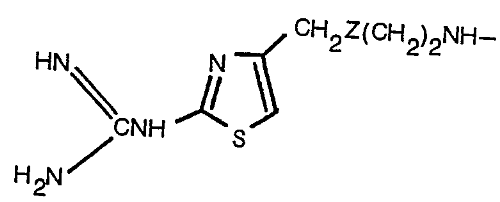

et $X^1$ est un groupement labile déplaçable par une amine de formule QS— (Q étant un radical alcoyle en $C_{1-4}$, aryle ou aralcoyle), avec un composé de formule (V):—

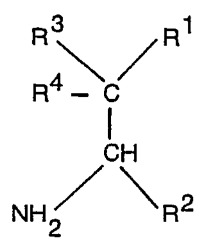
(V)

où $R^1$ et $R^2$ sont tels que définis par référence à la formula (I) et $R^3$ et $R^4$ sont des groupements alcoxy en $C_{1-4}$ ou représentent ensemble un atome d'oxygène et, facultativement, la conversion du composé de formule (I) ainsi obtenu en un sel.

11. Composition pharmaceutique comprenant un composé tel que défini dans l'une quelconque des revendications 1 à 8 et un excipient pharmaceutiquement acceptable.

**Revendications pour l'Etat contractant: AT**

1. Procédé pour préparer un composé de formule (I):—

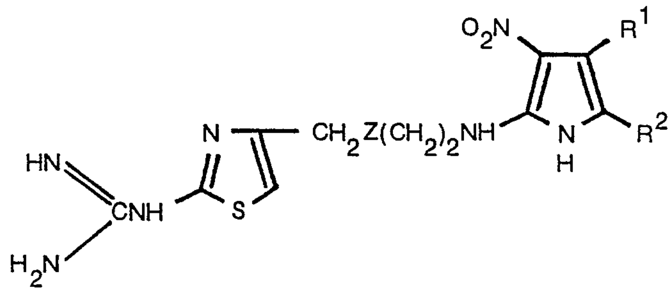

(I)

et ses sels d'addition d'acides pharmaceutiquement acceptables, où Z est du soufre ou un groupement méthylène et $R^1$ est de l'hydrogène; un radical alcoyle en $C_{1-4}$, un radical phényle ou phényl ($C_{1-4}$ alcoyle) facultativement substitué (les substituants étant un ou plusieurs groupements alcoyle en $C_{1-4}$ ou alcoxy en $C_{1-4}$ ou atomes d'halogène ou un groupement méthylènedioxy) ou un radical furyl-, thiényl- ou pyridyl- ($C_{1-4}$ alcoyle) facultativement substitué, les substituants étant un ou plusieurs groupements alcoyle en $C_{1-4}$ ou alcoxy en $C_{1-4}$ et $R^2$ est de l'hydrogène, un radical alcoyle en $C_{1-4}$ ou benzyle qui comprend soit

a) la réaction d'un composé de formule (II):—

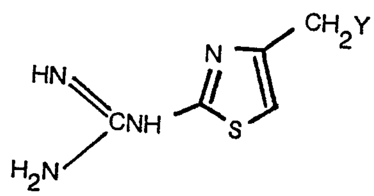

(II)

où Y est soit $Z(CH_2)_2NH_2$ soit, lorsque Z dans le composé de formule (I) est du soufre, un groupement labile déplaçable par un mercaptan, avec un composé de formule (III):—

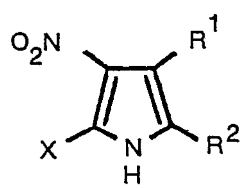

(III)

où X est un groupement labile déplaçable par une amine lorsque Y est $Z(CH_2)_2NH_2$ ou $HS(CH_2)_2NH$— lorsque Y est un groupement labile déplaçable par un mercaptan et $R^1$ et $R^2$ sont définis par référence à la formule (I) ou

(b) La réaction d'un composé de formule (IV):—

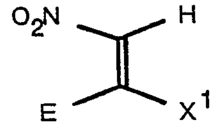

(IV)

où E est

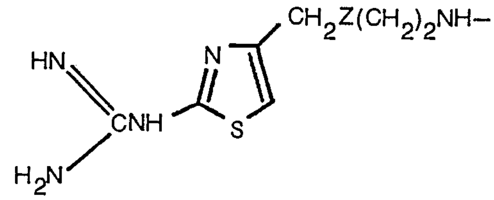

et $X^1$ est un groupe labile déplaçable par une amine de formule QS— (Q étant un radical alcoyle en $C_{1-4}$ aryle ou aralcoyle) avec un composè de formule (V):—

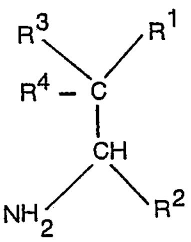

(V)

où $R^1$ et $R^2$ sont définis par référence à la formule (I) et $R^3$ et $R^4$ sont des groupements alcoxy en $C_{1-4}$ ou représentent ensemble un atome d'oxygène et, facultativement, la conversion en un sel d'un composé de formule (I) ainsi obtenu dans l'étape (a) ou dans l'étape (b).

2. Procédé tel que revendiqué dans la revendication 1 où $R^1$ dans le composé de la formule (I) est un groupement 3-méthylphényle, 3-méthoxyphényle, 3,4-diméthoxyphényle ou 3-chloro-phényle.

3. Procédé tel que revendiqué dans la revendication 1 où la revendication 2 où le groupement $R^1$ furyl-, thiényl- ou pyridyl ($C_{1-4}$ alcoyle) facultativement substitué dans le composé de formule (I) produit est un groupement 2-furyl-, 2-thiényl-, 3-pyridyl- ou 4-pyridyl- ($C_{1-4}$ alcoyle).

4. Procédé tel que revendiqué dans l'une quelconque des revendications 1 à 3 où $R^1$ dans le composé de formule (I) produit est un groupement 3-pyridyl-, 6-méthyl-3-pyridyl- ou 6-méthoxy-3-pyridyl- ($C_{1-4}$ alcoyle).

5. Procédé tel que revendiqué dans l'une quelconque des revendications 1 à 4 où $R^1$ dans le composé de formule (I) produit est de l'hydrogène.

6. Procédé tel que revendiqué dans l'une quelconque des revendications 1 à 5 où $R^2$ dans le composé formule (I) produit est de l'hydrogène.

7. Procédé tel que revendiqué dans l'une quelconque des revendications de 1 à 6 où Z dans le composé de formule (I) produit est du soufre.

8. Procédé tel que revendiqué dans la revendication 1 où le produit dans le composé de la formule (I) produit est le 2 - [2 - (2 - guanidino - 4 - thiazolylméthylthio) - éthylamino] - 3 - nitropyrrole, 2 - [2 - (2 - guanidino - 4 - thiazolylméthylthio) - éthylamino] - 4 - méthyl - 3 - nitropyrrole ou 2 - [2 - (2 - guanidino - 4 - thiazolylméthylthio) butylamino] - 3 - nitropyrrole.

**Patentansprüche für die Vertragsstaaten: BE CH DE FR GB IT LI LU NL SE**

1. Eine Verbindung der Formel (I)

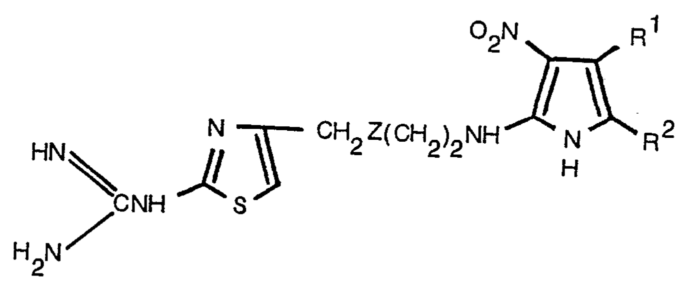

(I)

und pharmazeutisch verträgliche Säureadditionssalze davon, in der Z Schwefel oder Methylen ist und $R^1$ Wasserstoff, $C_{1-4}$-Alkyl, gegebenenfalls substituiertes Phenyl oder Phenyl-($C_{1-4}$-Alkyl), (wobei die Substituenten mindestens ein $C_{1-4}$-Alkyl- oder $C_{1-4}$-Alkoxyrest oder Halogenatom oder eine Methylendioxygruppe sind), oder gegebenenfalls substituiertes Furanyl-, oder Thienyl- oder Pyridyl-($C_{1-4}$-alkyl) ist, wobei die Substituenten mindestens ein $C_{1-4}$-Alkyl- oder $C_{1-4}$-Alkoxyrest sind, und $R^2$ Wasserstoff, $C_{1-4}$-Alkyl oder Benzyl ist.

2. Eine Verbindung gemäß Anspruch 1, wobei $R^1$ 3-Methylphenyl, 3-Methoxyphenyl, 3,4-Dimethoxyphenyl oder 3-Chlorphenyl ist.

3. Eine Verbindung gemäß Anspruch 1 oder Anspruch 2, wobei der gegebenenfalls substituierte Furanyl-, Thienyl- oder Pyridyl-($C_{1-4}$-alkyl)-Rest $R^1$ eine gegebenenfalls substituierte 2-Furanyl-, 2-Thienyl-, 3-Pyridyl- oder 4-Pyridyl- ($C_{1-4}$-alkyl)-Gruppe ist.

4. Eine Verbindung nach einem der Ansprüche 1 bis 3, wobei $R^1$ 3-Pyridyl-, 6-Methyl-3-pyridyl- oder 6-Methoxy-3-pyridyl-($C_{1-4}$-alkyl) ist.

5. Eine Verbindung nach einem der Ansprüche 1 bis 4, wobei $R^1$ Wasserstoff ist.

6. Eine Verbindung nach einem der Ansprüche 1 bis 5, wobei $R^2$ Wasserstoff ist.

7. Eine Verbindung nach einem der Ansprüche 1 bis 6, wobei Z Schwefel ist.

8. 2 - [2 - (2 - Guanidino - 4 - thiazolylmethylthio) - äthylamino] - 3 - nitropyrrol, 2 - [2 - (2 - Guanidino - 4 - thiazolylmethylthio) - äthylamino] - 4 - methyl] - 3 - nitropyrrol und 2 - [2 - (2 - Guanidino - 4 - thiazolylmethylthio) - butylamino] - 3 - nitropyrrol.

9. Ein Verfahren zur Herstellung einer Verbindung gemäß Anspruch 1, das Umsetzen einer Verbindung der Formel (II)

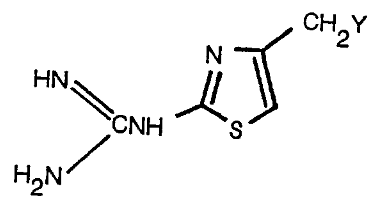

(II)

in der Y entweder $Z(CH_2)_2NH_2$ oder, wenn Z in der Verbindung der Formel (I) Schwefel bedeutet, eine durch Mercaptan ersetzbare austretende Gruppe darstellt, mit einer Verbindung der Formel (III)

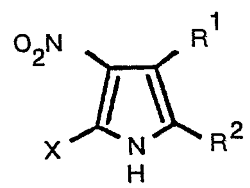

(III)

in der X eine durch ein Amin ersetzbare austretende Gruppe, wenn Y $Z(CH_2)_2NH_2$ bedeutet, oder $HS(CH_2)_2NH$— darstellt, wenn Y eine durch Mercaptan ersetzbare austretende Gruppe ist, und $R^1$ und $R^2$ wie in Verbindung mit Formel (I) definierte sind, und danach gegebenenfalls Umwandeln der so erhaltenen Verbindung der Formel (I) in ein Salz umfaßt.

10. Ein Verfahren zur Herstellung einer Verbindung gemäß Anspruch 1, das Umsetzung einer Verbindung der Formel (IV)

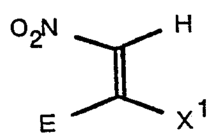

(IV)

in der E

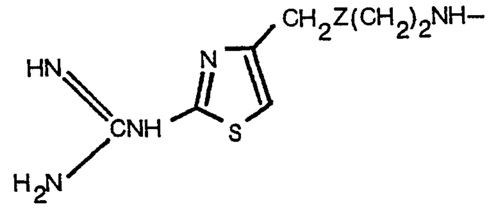

bedeutet und $X^1$ eine durch Amin der Formal QS— ersetzbare austretende Gruppe ist (Q bedeutet $C_{1-4}$-Alkyl, Aryl oder Aralkyl), mit einer Verbindung der Formel (V)

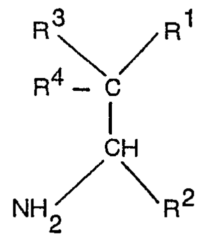

(V)

in der $R^1$ und $R^2$ wie in Verbindung mit Formel (I) definiert sind und $R^3$ und $R^4$ $C_{1-4}$-Alkoxyreste sind oder zusammen ein Sauerstoffatom bedeuten, und gegebenenfalls Umwandlung einer so erhaltenen Verbindung der Formel (I) in ein Salz umfaßt.

11. Arzneimittel, umfassend eine Verbindung gemäß einem der Ansprüche 1 bis 8 und einen pharmazeutisch verträglichen Träger.

**Patentansprüche für den Vertragsstaat: AT**

1. Verfahren zur Herstellung einer Verbindung der Formel (I)

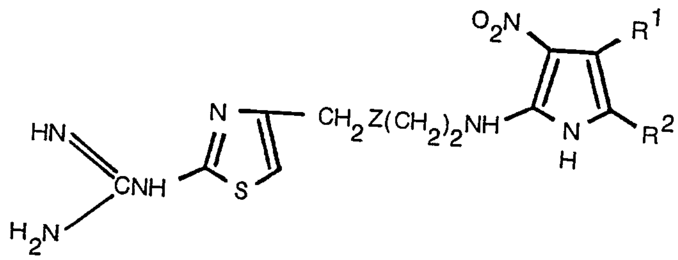

(I)

und pharmazeutisch verträglicher Säureadditionssalze davon, in der Z Schwefel oder Methylen ist und $R^1$ Wasserstoff, $C_{1-4}$-Alkyl, gegebenenfalls substituiertes Phenyl oder Phenyl-($C_{1-4}$-Alkyl), (wobei die Substituenten mindestens ein $C_{1-4}$-Alkyl- oder $C_{1-4}$-Alkoxyrest oder Halogenatom oder eine Methylendioxygruppe sind), oder gegebenenfalls substituiertes Furanyl-, oder Thienyl- oder Pyridyl-($C_{1-4}$-alkyl) ist, wobei die Substituenten mindestens ein $C_{1-4}$-Alkyl- oder $C_{1-4}$-Alkoxyrest sind, und $R^2$ Wasserstoff, $C_{1-4}$-Alkyl oder Benzyl ist, das entweder

a) Umsetzen einer Verbindung der Formel (I)

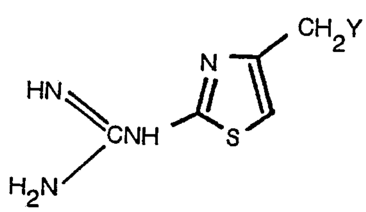

(II)

in der Y entweder $Z(CH_2)_2NH_2$ oder, wenn Z in der Verbindung der Formel (I) Schwefel bedeutet, eine durch Mercaptan ersetzbare austretende Gruppe darstellt, mit einer Verbindung der Formel (III)

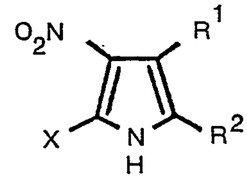

(III)

in der X eine durch ein Amin ersetzbare austretende Gruppe, wenn Y $Z(CH_2)_2NH_2$ bedeutet, oder $HS(CH_2)_2NH$— darstellt, wenn Y eine durch Mercaptan ersetzbare austretende Gruppe ist, und $R^1$ und $R^2$ wie in Verbindung mit Formel (I) definiert sind, oder

b) Umsetzung einer Verbindung der Formel (IV)

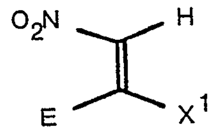

(IV)

in der E

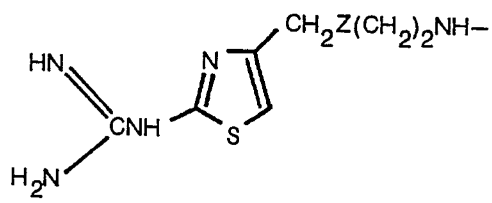

bedeutet und $X^1$ eine durch Amin der Formel QS— ersetzbare austretende Gruppe ist (Q bedeutet $C_{1-4}$-Alkyl, Aryl oder Aralkyl), mit einer Verbindung der Formel (V)

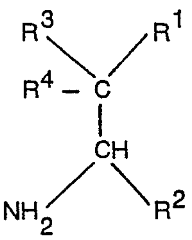

(V)

in der $R^1$ und $R^2$ wie in Verbindung mit Formel (I) definiert sind und $R^3$ und $R^4$ $C_{1-4}$-Alkoxyreste sind oder zusammen ein Sauerstoffatom bedeuten, und gegebenenfalls Umwandlung einer so in Stufe a) oder Stufe b) erhaltenen Verbindung der Formel (I) in ein Salz umfaßt.

2. Ein Verfahren nach Anspruch 1, wobei $R^1$ in der Verbindung der Formel I 3-Methylphenyl, 3-Methoxyphenyl, 3,4-Di-methoxyphenyl oder 3-Chlorphenyl ist.

3. Ein Verfahren nach Anspruch 1 oder Anspruch 2, wobei der gegebenenfalls substituierte Furanyl-, Thienyl- oder Pyridyl-($C_{1-4}$-alkyl)-Rest $R^1$ in der erzeugten Verbindung der Formel (I) eine gegebenenfalls substituierte 2-Furanyl-, 2-Thienyl-, 3-Pyridyl- oder 4-Pyridyl-($C_{1-4}$-alkyl)-Gruppe ist.

4. Ein Verfahren nach einem der Ansprüche 1 bis 3, wobei $R^1$ in der erzeugten Verbindung der Formel (I) 3-Pyridyl-, 6-Methyl-3-pyridyl- oder 6-Methoxy-3-pyridyl-($C_{1-4}$-alkyl) ist.

5. Ein Verfahren nach einem der Ansprüche 1 bis 4, wobei $R^1$ in der erzeugten Verbindung der Formel (I) Wasserstoff ist.

6. Ein Verfahren nach einem der Ansprüche 1 bis 5, wobei $R^2$ in der erzeugten Verbindung der Formel (I) Wasserstoff ist.

7. Ein Verfahren nach einem der Ansprüche 1 bis 6, wobei Z in der erzeugten Verbindung der Formel (I) Schwefel ist.

8. Ein Verfahren nach Anspruch 1, wobei das Produkt in der erzeugten Verbindung der Formel (I) 2 - [2 - (2 - Guanidino - 4 - thiazolylmethylthio) - äthylamino] - 3 - nitropyrrol, 2 - [2 - (2 - Guanidino - 4 - thiazolylmethylthio) - äthylamino] - 4 - methyl - 3 - nitropyrrol oder 2 - [2 - (2 - Guanidino - 4 - thiazolylmethylthio) - butylamino] - 3 - nitropyrrol ist.